(19) Europäisches Patentamt
European Patent Office
Office européen des brevets

(11) EP 3 805 450 B1

(12) **EUROPEAN PATENT SPECIFICATION**

(45) Date of publication and mention of the grant of the patent:
**31.05.2023 Bulletin 2023/22**

(21) Application number: **19811758.2**

(22) Date of filing: **01.08.2019**

(51) International Patent Classification (IPC):
***D06M 13/224*** (2006.01) ***A61F 13/15*** (2006.01)
***A61F 13/511*** (2006.01) ***D04H 1/541*** (2012.01)
***D06M 13/148*** (2006.01) ***D06M 13/165*** (2006.01)
***D06M 15/53*** (2006.01) ***A61K 8/02*** (2006.01)
***A61K 8/34*** (2006.01) ***A61K 8/37*** (2006.01)
***A61K 8/49*** (2006.01) ***A61K 8/86*** (2006.01)
***A61K 8/92*** (2006.01) ***A61L 15/26*** (2006.01)
***A61L 15/34*** (2006.01) ***A61L 15/48*** (2006.01)
***D04H 1/4391*** (2012.01) ***D06M 13/17*** (2006.01)
***D06M 15/03*** (2006.01) ***A61K 8/60*** (2006.01)

(52) Cooperative Patent Classification (CPC):
**D04H 1/43918; A61K 8/0208; A61K 8/345; A61K 8/375; A61K 8/60; A61K 8/86; A61L 15/26; A61L 15/34; A61L 15/48; D04H 1/5412; D06M 13/148; D06M 13/17; D06M 13/2243; D06M 15/03; D06M 15/53;** (Cont.)

(86) International application number:
**PCT/JP2019/030168**

(87) International publication number:
**WO 2019/230993 (05.12.2019 Gazette 2019/49)**

(54) **SYNTHETIC FIBER AND FIBER TREATMENT AGENT**

KUNSTFASER UND FASERBEHANDLUNGSMITTEL

FIBRE SYNTHÉTIQUE ET AGENT DE TRAITEMENT DE FIBRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.04.2021 Bulletin 2021/15**

(73) Proprietor: **TOYOBO CO., LTD.**
**Osaka-shi**
**Osaka 5300001 (JP)**

(72) Inventors:
- **JIZODO, Shinichi**
  **Osaka 530-8230 (JP)**
- **NOGUCHI, Shuji**
  **Osaka 530-8230 (JP)**
- **YAMAMOTO, Shuhei**
  **Osaka 530-8230 (JP)**

(74) Representative: **Müller-Boré & Partner Patentanwälte PartG mbB**
**Friedenheimer Brücke 21**
**80639 München (DE)**

(56) References cited:
**EP-A1- 2 055 314** **JP-A- 2009 275 017**
**JP-A- 2012 001 458** **JP-A- 2012 057 275**
**JP-A- 2017 014 135** **JP-A- 2017 081 907**

- **DATABASE WPI Week 201011 Thomson Scientific, London, GB; AN 2010-B09880 XP002805666, & JP 2010 018559 A (TOYOBO KK) 28 January 2010 (2010-01-28)**
- **DATABASE WPI Week 201637 Thomson Scientific, London, GB; AN 2016-29493W XP002805667, & WO 2016/076089 A1 (TOYOBO CO LTD) 19 May 2016 (2016-05-19)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE WPI Week 201224 Thomson Scientific, London, GB; AN 2012-D38845 XP002805668, & JP 2012 057275 A (DAIWABO HOLDINGS CO LTD) 22 March 2012 (2012-03-22)**

(52) Cooperative Patent Classification (CPC): (Cont.)
D04H 1/5414; D04H 1/5416; D06M 2101/20; D06M 2101/32

## Description

### Technical Field

Techniques concerning synthetic fiber, a fiber treatment agent, and use of the synthetic fiber and fiber treatment agent are disclosed.

### Background Art

Synthetic fiber is superior to natural fiber in properties such as mechanical characteristics and chemical resistance. Thus, synthetic fiber, usually in the form of non-woven fabric, is used in top sheets for, for example, liquid-impregnated sheets such as wipes, pre-moistened wipes, and facial masks; cosmetic or medical patches; and various hygiene products, such as paper diapers and sanitary napkins.

However, synthetic fiber, in particular, synthetic fiber that contains a polyolefin-based resin, such as polyethylene resin or polypropylene resin (or "polyolefin-based fiber"), has low hydrophilicity and is hydrophobic, compared with cotton (natural fiber) and rayon (cellulose-based fiber). Thus, the use of synthetic fiber in a top sheet of liquid-impregnated sheets or hygiene products requires an improvement in the hydrophilicity of synthetic fiber. There are reports on various methods for improving the hydrophilicity of synthetic fiber. One of the methods treats fiber with a lactate-containing fiber treatment agent, and adheres (poly)glycerol to the surface of the fiber (PTL 1).

Additionally, top sheets for cosmetic or medical patches or hygiene products, for example, may be required to have moisturizing properties, in order to relieve the dryness of part of the skin that comes in contact with the top sheets. PTL2 discloses a liquid composition containing a predeterminded amount of (A) mannosylerythritol lipid-B; (B) glycerin; (C) polyhydric alcohol (except for glycerin); (D) glycyrrhizinate; and (e) water.

### Citation List

#### Patent Literature

PTL 1: JP2012-57275
PTL 2 : WO2016/076089A

### Summary of Invention

#### Technical Problem

An object is to provide a synthetic fiber that exhibits improved properties of conventional synthetic fiber, and a fiber treatment agent for improving the properties. For example, an object is to provide a synthetic fiber that exhibits improved hydrophilicity, a synthetic fiber that exhibits improved moisturizing properties, and a fiber treatment agent therefor. Solution to Problem

The present inventors found that treating synthetic fiber with a fiber treatment agent that contains a polyglycerol compound selected from the group consisting of polyglycerols, polyglycerol fatty acid esters, and alkylene oxide adducts of polyglycerols; and a mannosylerythritol lipid (MEL) can impart properties such as moisturizing properties and hydrophilicity to the synthetic fiber. Additionally, while it is suggested that adding a surfactant with a lipopeptide structure such as surfactin, a sophorolipid, a rhamnolipid, or MEL to a fiber treatment agent that substantially contains no lactate leads to a significant decrease in the hydrophilicity-imparting action of the fiber treatment agent (Comparative Examples 11 to 16 of PTL 1), the inventors found that adding MEL to a glycerol compound-containing fiber treatment agent can improve the hydrophilicity-imparting action of the fiber treatment agent even though the fiber treatment agent contains substantially no lactate.

#### Advantageous Effects of Invention

In an embodiment, a synthetic fiber excellent in hydrophilicity is provided. In an embodiment, a synthetic fiber with moisturizing properties is provided. In an embodiment, a fiber treatment agent that imparts hydrophilicity to synthetic fiber is provided. In an embodiment, a fiber treatment agent that imparts hydrophilicity and moisturizing properties to synthetic fiber is provided. In an embodiment, a fiber treatment agent whose hydrophilicity-imparting action has been increased by MEL is provided.

Description of Embodiments

The following mainly describes the above-described typical embodiments.

The synthetic fiber contains component (a) and component (b) both adhered to the surface of the fiber. Component (a) is a mannosylerythritol lipid (MEL). Component (b) is at least one polyglycerol compound selected from the group consisting of polyglycerols, polyglycerol fatty acid esters, and alkylene oxide adducts of polyglycerols

Component (a): MEL

The structure of MEL is illustrated in Formula (1).

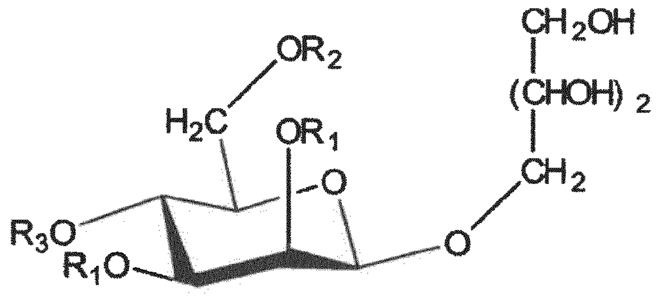

Formula (1)

In Formula (1), substituents $R_1$ may be identical or different and represent an aliphatic acyl group having 2 to 24 carbon atoms, and substituents $R_2$ and $R_3$ may be identical or different and represent an acetyl group or a hydrogen atom.

In Formula (1), substituents $R_1$ may be identical or different and represent an aliphatic acyl group having 2 to 24, preferably 2 to 20 or 4 to 24, more preferably 4 to 18, and still more preferably 6 to 14 carbon atoms.

The MEL is classified into the following four types: mannosylerythritol lipid A (MEL-A), mannosylerythritol lipid B (MEL-B), mannosylerythritol lipid C (MEL-C), and mannosylerythritol lipid D (MEL-D), according to whether the mannose has an acetyl group at position 4 or at position 6.

MEL-A is represented by Formula (1) wherein substituents $R_2$ and $R_3$ are both an acetyl group. MEL-B is represented by Formula (1) wherein substituent $R_2$ is an acetyl group, and substituent $R_3$ is a hydrogen atom. MEL-C is represented by Formula (1) wherein substituent $R_2$ is a hydrogen atom, and substituent $R_3$ is an acetyl group. MEL-D is represented by Formula (1) wherein substituent $R_2$ and $R_3$ are both a hydrogen atom.

The number of carbon atoms of each substituent $R_1$ in MEL-A to MEL-D varies depending on the number of carbon atoms of fatty acid that constitutes a triglyceride, which is a type of lipid contained in a MEL production medium; and the degree of assimilation of fatty acid by MEL-producing microorganisms used. When the triglyceride has an unsaturated fatty acid residue, it is also possible to incorporate the unsaturated fatty acid residue as substituent $R_1$, unless the MEL-producing microorganisms assimilates the double bond portion of the unsaturated fatty acid. As is clear from the above explanation, the obtained MEL is typically a mixture of compounds that differ in terms of the fatty acid residue portion of substituents $R_1$.

Triacylated MEL

The structure of triacylated MELs (i.e., triacyl mannosylerythritol lipids, which are also called "triacyl MELs") is illustrated in Formulas (2) and (3).

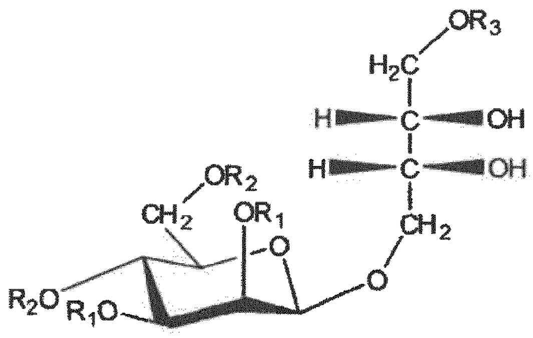

Formula (2)

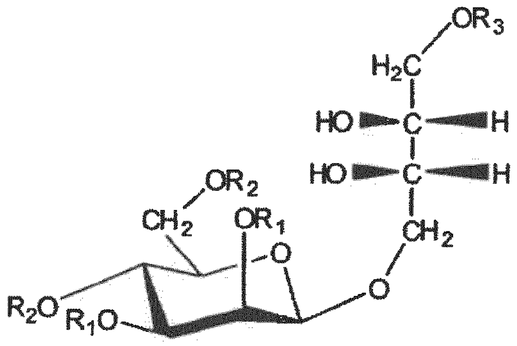

Formula (3)

In Formulas (2) and (3), substituents $R_1$ may be identical or different and represent an aliphatic acyl group having 2 to 24 carbon atoms; substituents $R_2$ may be identical or different and represent an acetyl group or a hydrogen atom; and substituents $R_3$ may be identical or different and represent an aliphatic acyl group having 2 to 24 carbon atoms.

In Formulas (2) and (3), substituents $R_1$ may be identical or different and represent an aliphatic acyl group having 2 to 24, preferably 2 to 20 or 4 to 24, more preferably 4 to 18, and still more preferably 6 to 14 carbon atoms; substituents $R_2$ may be identical or different and represent a hydrogen atom or acetyl group; and substituents $R_3$ represent an aliphatic acyl group having 2 to 24, preferably 2 to 20 or 4 to 24, more preferably 4 to 18, and still more preferably 6 to 14 carbon atoms.

As with MEL, triacyl MELs are also classified into the following four types of triacyl MELs: triacyl MEL-A, triacyl MEL-B, triacyl MEL-C, and triacyl MEL-D, according to whether the mannose has an acetyl group at position 4 or at position 6.

A triacylated MEL can be obtained, for example, from a culture solution of MEL-producing microorganisms. A triacylated MEL can also be produced by reacting MEL with various plant oils by using an enzyme.

MEL is, for example, at least one member selected from the group consisting of mannosylerythritol lipid A (MEL-A), mannosylerythritol lipid B (MEL-B), mannosylerythritol lipid C (MEL-C), mannosylerythritol lipid D (MEL-D), triacylated MEL-A, triacylated MEL-B, triacylated MEL-C, and triacylated MEL-D.

A preferable MEL is at least one member selected from the group consisting of MEL-A and MEL-B, with MEL-B being more preferable. MEL-B with a structure illustrated in Formula (4) or (5) is preferable.

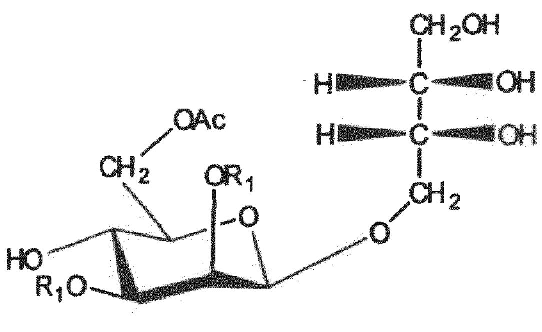

Formula (4)

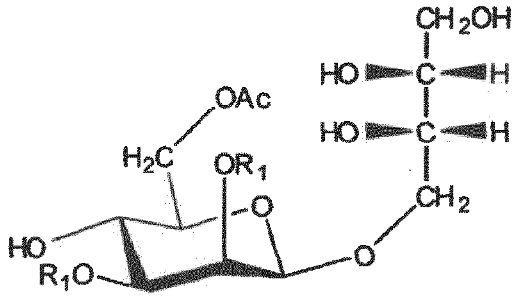

Formula (5)

In Formulas (4) and (5), substituents $R_1$ may be identical or different and represent an aliphatic acyl group having 4 to 24 carbon atoms.

In Formulas (4) and (5), substituents $R_1$ may be identical or different and represent an aliphatic acyl group having 4 to 24, preferably 4 to 18, and still more preferably 6 to 14 carbon atoms.

MELs may be used singly, or in a combination of two or more.

The MEL for use in the present invention can be any MEL; and is, for example, a commercially available MEL, or a MEL produced by a known production method. For example, MELs (MEL-A, MEL-B, and MEL-C) can be produced by culturing a microorganism that can produce a MEL (MEL-producing microorganism), such as *Pseudozyma antarctica* (NBRC 1073), *Pseudozyma tsukubaensis, Pseudozyma hubeiensis, Pseudozyma graminicola,* or *Pseudozyma* sp., in accordance with an ordinary method.

Component (b): polyglycerol Compound

Component (b) is at least polyglycerol compound selected from the group consisting of polyglycerols, polyglycerol fatty acid esters, and alkylene oxide adducts of polyglycerols As used herein, "polyglycerol" refers to either a polyglycerol, or a mixture of a monoglycerol and a polyglycerol. Polyglycerols having an average degree of polymerization of 2 to 30 are preferable for use. Polyglycerols having an average degree of polymerization of 2 to 15 are more preferable for use. Specific examples of polyglycerols include diglycerol, tetraglycerol, hexaglycerol, octaglycerol, and decaglycerol. polyglycerol fatty acid esters refer to an ester of a polyglycerol and a fatty acid; and include monoesters, diesters, and triesters. A fatty acid ester of a polyglycerol contains a fatty acid component derived from a fatty acid having, for example, 10 to 22, and preferably 12 to 18 carbon atoms. The fatty acid may be saturated or unsaturated. Specific examples of such fatty acids include saturated fatty acids, such as capric acid, lauric acid, tridecylic acid, myristic acid, pentadecylic acid, palmitic acid, heptadecylic acid, stearic acid, isostearic acid, nonadecanoic acid, arachidic acid, and behenic acid; and unsaturated fatty acids, such as oleic acid. Component (b) for use is preferably a polyglycerol mono-fatty acid ester in which a fatty acid having 10 to 22 carbon atoms is ester-linked to a polyglycerol with a degree of polymerization of 2 to 15.

Component (b) for use is particularly preferably a polyglycerol monolaurate in which a fatty acid having 12 carbon atoms (lauric acid) is ester-linked to a polyglycerol with a degree of polymerization of 6 to 12.

Polyglycerols for use in polyglycerol fatty acid esters are typically produced by a glycerol polymerization method in which a (mono)glycerol is subjected to dehydration condensation to increase the degree of polymerization of a polyglycerol by using a glycerol-related substance, such as (mono)glycerol, glycidol, or epichlorohydrin as a starting material; or a production method such as a glycidol method, an epichlorohydrin method, or a diglycerol crosslink method. When a polyglycerol is produced by any of these production methods, not only a linear polyglycerol with a target degree of polymerization but also cyclic polyglycerols having a 6-membered ring or 8-membered ring are formed as by-products. Thus, the polymerized polyglycerols may contain these by-products. Additionally, polyglycerols with a low degree of polymerization, which is different from the target degree of polymerization, are also formed as by-products; and may be contained in the polymerized polyglycerols. In particular, cyclic polyglycerols are considered to have low hydrophilicity, and be highly irritating to the human body. Thus, the content of cyclic polyglycerols is preferably low.

The average degree of polymerization of a polyglycerol refers to the average degree of polymerization determined from a hydroxy value used in commonly distributed polyglycerols. The content of cyclic forms present in a polyglycerol can be analyzed with, for example, a liquid chromatography-mass spectrometer (LC/MS).

The average number of moles of alkylene oxide added in an alkylene oxide adduct of a polyglycerol is, for example 2 to 30, preferably 4 to 24, and more preferably 8 to 20. The alkylene oxide for use in alkylene oxide adducts of polyglycerols is, for example, an alkylene oxide having 2 to 4 carbon atoms. Specific examples of alkylene oxides include ethylene oxide, propylene oxide, and butylene oxide. The alkylene oxide adduct of a polyglycerol for use is preferably an alkylene oxide adduct of diglycerol obtained by adding an alkylene oxide to diglycerol, and particularly preferably a propylene oxide adduct of diglycerol. The average number of moles of propylene oxide added in a propylene oxide adduct of polyglycerol is, for example, within the ranges of the average number of moles described above; and is

preferably 9 to 14.

The amount of component (a) adhered to the surface of fibers of synthetic fiber may be, for example, 0.05 mass% or more, 0.1 mass% or more, or 0.3 mass% or more; and for example, 10 mass% or less, 5 mass% or less, 3 mass% or less, or 1 mass% or less based on the mass of the synthetic fiber. In an embodiment, the amount of component (a) adhered to the fibers is, for example, 0.05 to 10 mass%, preferably 0.1 to 5 mass%, and more preferably 0.3 to 3 mass%.

The amount of component (b) adhered to the surface of fibers of synthetic fiber can be any amount that can bring about the effects, and may be, for example 0.01 mass% or more, 0.05 mass% or more, or 0.1 mass% or more; and, for example, 1 mass% or less, 0.8 mass% or less, or 0.5 mass% or less, based on the mass of the synthetic fiber. In an embodiment, the amount of component (b) adhered to fibers is, for example, 0.01 to 1 mass%, preferably 0.05 to 0.8 mass%, and more preferably 0.1 to 0.5 mass%. The amount component (a) or (b) adhered to fibers refers to the amount of adhered component (a) or (b) based on the mass of an ultimately obtained, dry synthetic fiber.

The synthetic fiber according to an embodiment is a synthetic fiber to whose surface a fiber treatment agent is adhered. The fiber treatment agent contains (a) a mannosylerythritol lipid (MEL), and (b) at least one polyglycerol compound selected from the group consisting of polyglycerols, polyglycerol fatty acid esters, and alkylene oxide adducts of polyglycerols, wherein the total mass of component (a) and component (b) is 40 mass% or more based on the entire fiber treatment agent, and the mass of component (a) is 33 to 99.5 mass% based on the total mass of component (a) and component (b).

In an embodiment, the fiber treatment agent that contains component (a) and component (b) is prepared such that component (a) is present in an amount of, for example, 33 to 99.5 mass%, preferably 50 to 90 mass%, and more preferably 60 to 80 mass%, based on the total mass of component (a) and component (b) taken as 100 mass%. In an embodiment, the amount of component (b) may be any amount, as long as the amount of component (a) falls within the ranges above. For example, when component (a) is present in an amount of 33 mass%, component (b) is present in an amount of 67 mass%; when component (a) is present in an amount of 67 mass%, component (b) is present in an amount of 33 mass%. The amount of component (a) and the amount of component (b) that fall within the ranges are advantageous in terms of hydrophilicity imparted to the synthetic fiber, moisturizing properties, and fiber processability (e.g., the properties of reducing static electricity in the carding process during fiber processing).

In an embodiment, the total mass of component (a) and component (b) in the fiber treatment agent accounts for, for example, 40 mass% or more, 50 mass% or more, 60 mass% or more, 70 mass% or more, 80 mass% or more, 85 mass% or more, 90 mass% or more, 95 mass% or more, or 97 mass% or more of the entire treatment agent. When a fiber treatment agent is prepared by mixing component (a) and component (b) with one or more other components, the other components may be selected from components added to a fiber treatment agent, according to the desired hydrophilicity, moisturizing properties, fiber processability, and other properties of the synthetic fiber to be obtained. The fiber treatment agent may contain other hydrophilic components, other moisturizing components, fiber-processability-improving components, and the like components, as long as the effects of the present invention are not impaired. The fiber treatment agent contains no salt of lactic acid with a metal (e.g., potassium lactate, sodium lactate, magnesium lactate, calcium lactate, and aluminum lactate).

Components that can be contained in the fiber treatment agent, other than component (a) and component (b), include components typically used in fiber treatment agents. Specific examples include various tocopherols, such as α-tocopherol, β-tocopherol, γ-tocopherol, and δ-tocopherol; various carotenoids, such as β-carotene, lycopene, lutein, and astaxanthin; lipid-soluble antioxidants, such as ubiquinone (ubiquinol, ubidecarenone, CoQ10), α-lipoic acid (lipoic acid, thioctic acid), BHT (dibutyl hydroxy toluene), and BHA (butyl hydroxyanisol); various catechins, such as ascorbic acid, isoascorbic acid, catechin, catechin gallate, epigallocatechin gallate, epigallocatechin, epicatechin gallate, epicatechin, gallocatechin gallate, and gallocatechin; various flavonoids, such as anthocyanin, tannin, and quercetin (including various glycosides such as rutin and quercitrin, and those enzymatically or chemically modified, such as enzymatically modified isoquercitrin), myricitrin, myricetin, and isoflavone; various phenolic acids, such as chlorogenic acid, ellagic acid, and curcumin; water-soluble antioxidants, such as polyphenols, including apple polyphenol, and cocoa mass polyphenol; plant-derived essential oils, such as phytoncide, hiba oil, and hinokitiol; aromatic functional agents, such as herb oil; other deodorizers; antimicrobial agents; allergen deactivators; moisture-absorbing and heat-generating agents; and far-infrared heat-retaining agents.

Alternatively, the fiber treatment agent may also contain a surfactant other than component (a). When the fiber treatment agent contains a surfactant other than component (a), the surfactant other than component (a) may be one or more surfactants selected from the following known surfactants: nonionic surfactants, such as sugar ester surfactants (or "polyhydric alcohol ester surfactants"), fatty acid ester surfactants, alcohol surfactants, alkyl phenol surfactants, polyoxyethylene-polyoxypropylene block polymer surfactants, alkyl amine surfactants, bisphenol surfactants, polyaromatic surfactants, silicone-based surfactants, fluorine-based surfactants, and plant oil surfactants; anionic surfactants, such as sulfate surfactants, sulfonate surfactants, carboxylic acid surfactants, and phosphate surfactants; cationic surfactants, such as ammonium surfactants and benzalkonium surfactants; and ampholytic surfactants, such as betaine surfactants, and glycine surfactants.

In an embodiment, it is preferred that a biosurfactant, which is a surfactant biologically derived from a microorganism (however, other than component (a)), be added to the fiber treatment agent. This is because such a biologically derived surfactant is considered to be less irritating to the skin, in the same manner as component (a). Biosurfactants include surfactants with a lipopeptide structure (which are also referred to as amino acid surfactants or acyl peptide surfactants), glycolipid biosurfactants such as trehalose lipids and cellobiose lipids (which are also referred to as glycosurfactants), phospholipid-based biosurfactants, fatty acid-based biosurfactants, and polymer compound-based biosurfactants. These biosurfactants may be used singly, or in a combination of two or more.

In addition to the above-described components typically used in the fiber treatment agent, the fiber treatment agent may contain various functional agents usable as cosmetics as components other than components (a) and (b), to the degree that the effects of the present invention are not impaired. Examples of such functional agents include functional agents that provide an anti-aging effect by increasing the synthesis of hyaluronic acid or exhibiting cell activation; functional agents that exhibit a skin-whitening effect by inhibiting and reducing the activity of tyrosinase to reduce the formation of melanin; functional agents that exhibit a moisturizing and emollient effect on the skin by maintaining the skin moisture; other functional agents that have a UV protection and UV care effect; functional agents that have an anti-inflammatory effect; and functional agents that have an irritation-reducing effect. For example, chitin, chitosan, and derivatives derived from these chitin or chitosan (e.g., carboxymethyl chitin and carboxymethyl chitosan) are functional agents usable in cosmetics, and exhibit antimicrobial action.and moisturizing action when added to the fiber treatment agent. Adding various functional agents usable in cosmetics to the fiber treatment agent enables the synthetic fiber or fiber assembly according to the present invention to increase the added value of the products that come and stay in direct contact with the skin for a long period of time, such as top sheets and cosmetic-impregnated sheets of hygiene products (e.g., paper diapers and sanitary napkins).

The fiber treatment agent is preferably used such that the above-stated amount of component (a) adhered to fibers and the above-stated amount of component (b) adhered to fibers are achieved.

The synthetic fiber may be, for example, made of thermoplastic resin. Specific examples of thermoplastic resins include polyolefin-based resins, such as polyethylene (including high-density, low-density, and linear low-density polyethylenes), polypropylene, polybutene, polybutylene, polymethyl pentene resin, polybutadiene, ethylene-based copolymers (e.g., ethylene-$\alpha$-olefin copolymers), propylene-based copolymers (e.g., propylene-ethylene copolymers), ethylene-vinyl alcohol copolymers, ethylene-vinyl acetate copolymers, ethylene-(meth)acrylic acid copolymers, and ethylene-methyl (meth)acrylate copolymers; polyester resins, such as polyethylene terephthalate, polybutylene terephthalate, polytrimethylene terephthalate, polyethylene naphthalate, polylactic acid, polybutylene succinate, and copolymers thereof; polyamide-based resins, such as nylon 66, nylon 12, and nylon 6; and engineering plastics, such as acrylic-based resins, polycarbonate, polyacetal, polystyrene, cyclic polyolefin, mixtures thereof, and elastomer-based resins thereof.

The synthetic fiber may be in any form. For example, the synthetic fiber may be a single-component fiber composed of a single resin, or of a mixture of multiple resins; or a composite fiber composed of two or more components. The composite fiber may be, for example, a core-in-sheath composite fiber, an eccentric core-in-sheath composite fiber, a side-by-side composite fiber, an islands-in-the-sea composite fiber, or a segmented composite fiber in which resin components like pulps of citrus fruits are alternately arranged. The shape of the cross-section of the synthetic fiber can also be any shape. Thus, the synthetic fiber may be a typically used synthetic fiber that has a cross-section of circular shape (e.g., a true circle), a synthetic fiber that has a cross-section of non-circular shape, or a synthetic fiber that has a cross-section of irregular shape. Examples of the shape of the cross-section of irregular shape of a fiber include polygonal shapes, oval shapes, flattened shapes, so-called multi-lobed shapes that have multiple branching parts on the surface of fiber (specifically, a multi-lobed shape with 3 to 32 leaves), star shapes, C shapes, Y shapes, W shapes, crisscross shapes, and hash shapes. Additionally, the synthetic fiber may be a "solid fiber" that has no continuous void portion in the longitudinal direction in the fiber cross-section, or a "hollow fiber" that has one or more continuous void portions in the longitudinal direction in the fiber cross-section, regardless of whether the synthetic fiber is a single-component fiber or a composite fiber; and/or regardless of whether the shape of the fiber cross-section is circular or irregular, as described above.

When the fiber is a composite fiber that has a core component and a sheath component (including fiber with an eccentric structure), it is preferable to select resins that constitute the sheath component and the core component so as to satisfy "the melting point of the sheath component $\leq$ "the melting point of the core component -10°C." Such a combination provides a heat-adhesive composite fiber whose sheath component is usable as a heat-adhesive component.

The sheath component for use in the core-in-sheath composite fiber includes resins with a low melting point, such as polyethylenes (which include not only the resins formed from ethylene derived from crude oil such as naphtha, but also resins formed from raw materials derived from natural products, or resins formed by polymerizing raw materials derived from biomass (e.g., high-density polyethylene, low-density polyethylene, and linear low-density polyethylene), and polyethylene polymerized using a known catalyst such as a Ziegler-Natta catalyst or metallocene catalyst; however, the resins are not limited thereto), ethylene-based copolymers, propylene-based copolymers, copolymerized polyesters,

polybutylene succinate, and polybutylene succinate adipate (which is also referred to as "polybutylene succinate adipate"). Preferable combinations of the core and the sheath in the core-in-sheath composite fiber include a combination of polypropylene and high-density polyethylene, a combination of polypropylene and low-density polyethylene, a combination of polypropylene and linear low-density polyethylene, a combination of polyethylene terephthalate and high-density polyethylene, a combination of polyethylene terephthalate and low-density polyethylene, a combination of polyethylene terephthalate and linear low-density polyethylene, a combination of polypropylene and an ethylene-propylene copolymer, a combination of polyethylene terephthalate and an ethylene-propylene copolymer, a combination of polylactic acid and polyethylene, a combination of polylactic acid and polybutylene succinate, and a combination of polylactic acid and polybutylene succinate adipate.

When the synthetic fiber is prepared as a heat-adhesive core-in-sheath synthetic fiber, the ratio of the core component to the sheath component (volume ratio) is preferably 2/8 to 8/2 from the standpoint of the spinnability, adhesiveness, and processability of fiber; and advantageously 3/7 to 7/3 from the standpoint of the ease of passing the carding machine or the heat-adhesiveness of fiber.

The fiber treatment agent enables the synthetic fiber to have excellent hydrophilicity. Thus, a hydrophilicity-imparting effect is notably achieved in a fiber whose surface is composed of a hydrophobic thermoplastic resin; in particular, a fiber whose surface is composed of polyethylene, polypropylene, copolymerized polyester, an ethylene-based copolymer, and/or a propylene-based copolymer.

The fiber has a fineness of preferably 0.3 to 20 dtex, and more preferably 0.3 to 10 dtex. The fineness of synthetic fiber can be suitably selected according the intended use of the fiber. For example, a synthetic fiber for use in the top sheet of a hygiene product preferably has a fineness of 0.5 to 8 dtex, more preferably 0.7 to 7 dtex, and particularly preferably 1 to 7 dtex. A synthetic fiber for use in wipes or pre-moistened wipes preferably has a fineness of 0.5 to 10 dtex. A synthetic fiber for use in cosmetic-impregnated sheets such as facial masks, and base cloth for cosmetic or medical patches preferably has a fineness of 0.3 to 5 dtex. A synthetic fiber that has a fineness within these ranges is advantageous in terms of, for example, fiber strength, and flexibility of the synthetic fiber in the form of fiber assembly (e.g., non-woven fabric).

In an embodiment, the synthetic fiber can be obtained, for example, by treating a synthetic fiber with a fiber treatment agent that contains component (a) and component (b). In an embodiment, the synthetic fiber can also be obtained by first treating a synthetic fiber with component (b), and then treating with component (a).

For example, a resin, which is a starting material of a synthetic fiber to be treated, is molten-spun with a known molten spinning machine, using a suitable spinning nozzle at a suitable spinning temperature according to the form of fiber to be obtained. Subsequently, spun filaments (undrawn yarn) are optionally drawn. The drawing method and drawing conditions for drawing are not particularly limited; and can be suitably determined according to, for example, the type of resin, the combination of resins if the fiber is a composite fiber, and the properties of the fiber to be obtained. For example, spun filaments are drawn in warm water, hot air, or a heat medium at a drawing temperature of 60 to 110°C, with a 2.0-to 8.0-fold draw ratio. The drawing method is not particularly limited, and can be a known drawing method, such as wet-drawing performed in a high-temperature liquid such as warm water or hot water with heating; dry-drawing performed in a high-temperature gas, or by heating with a high-temperature metallic roll; or water-vapor drawing performed by heating fibers with water vapor at 100°C or higher, under ordinary pressure or increased pressure.

After the filaments are drawn, the fiber treatment agent is adhered to the filaments. Specifically, a solution obtained by diluting the fiber treatment agent with water or another solvent ("treatment solution" below) is adhered to the surface of the obtained drawn filaments, and the filaments are dried to allow water (or another solvent) to evaporate from the adhered treatment solution. Due to this operation, the fiber treatment agent adheres to the dried filaments. The method for adhering the treatment solution to the surface of fibers is not particularly limited. For example, the treatment solution can be adhered by a known method such as a spraying method, an impregnation method, or a touch roll method. The dried filaments are optionally cut to a predetermined length; and provided as short fiber or staple fiber with a fiber length of about 2 to 100 mm, or long fiber (continuous fiber). The filaments may be treated with the fiber treatment agent after cut to a desired length.

The drawn filaments are optionally provided with crimps with the number of crimps of 10 to 25/25 mm, and a crimp percentage of about 8 to 25% using a fiber-crimping machine. When crimping is applied, it is preferable to adhere the treatment solution to filaments before crimping, or simultaneously with crimping.

The synthetic fiber according to the present invention is not limited to those obtained by adhering the fiber treatment agent in a fiber production stage, but also includes those obtained by adhering the fiber treatment agent to the synthetic fiber in a fiber assembly production stage using a synthetic fiber. For example, the synthetic fiber according to the present invention can also be obtained by a method of adhering a treatment agent to the surface of at least part of the synthetic fibers that constitute a fiber assembly, such as a method in which a fiber assembly (e.g., non-woven fabric) is obtained by a method described later from an untreated synthetic fiber or synthetic fiber to which another fiber treatment agent is adhered, and then the fiber assembly is sprayed with a treatment agent prepared by adjusting the amount of components (a) and (b); or a method in which the fiber assembly is impregnated with the treatment agent.

Alternatively, in the stage before the synthetic fiber is formed into a fiber assembly, the fiber treatment agent may be adhered (for example, to a fibrous web for preparing a non-woven fabric, a woven fabric obtained by spinning fiber, or spun yarn for knitting).

The synthetic fiber obtained by the method as described above (an untreated synthetic fiber or a synthetic fiber to which another fiber treatment agent is adhered when the fiber treatment agent is adhered in the fiber assembly production stage) is processed into a known fiber assembly, such as a knitted fabric, net-like product, or non-woven fabric, for use. The fiber assembly contains the synthetic fiber according to the present invention in an amount of, for example, 50 mass% or more, 75 mass% or more, preferably 90 mass% or more, and more preferably 100 mass%. In particular, the synthetic fiber according to the present invention is preferably used in preparing a non-woven fabric. The non-woven fabric is produced by preparing a fibrous web, and then adhering and/or entangling the fibers to integrate them. The form of the fibrous web is not particularly limited, and may be any fibrous web, such as a parallel web, semi-random web, or cross web formed of staple fibers; a wet-laid web or air-laid web formed of short fibers; a spun-bond web or melt-blown web formed of long fibers; or a fibrous web obtained by electrospinning (which is also referred to as "electrostatic spinning"). A non-woven fabric for applications in which flexibility and texture are important is preferably prepared from a web formed of staple fibers.

In the production of a non-woven fabric, the method for integrating the fibers of a fibrous web is not particularly limited. For example, when the fibers are heat-adhesive core-in-sheath composite fibers, or when the fibers constitute a non-woven fabric together with heat-adhesive fibers (single-component fibers or core-in-sheath composite fibers), the fibers may be integrated by thermal bonding such as hot-air spray-coating or thermal embossing. Alternatively, the fibers may also be integrated by mechanical entangling, such as needle-punching or hydroentanglement.

In an embodiment, when the synthetic fiber is a heat-adhesive core-in-sheath composite fiber, it is preferred that a non-woven fabric be prepared using a fibrous web that contains the fiber of the present invention in an amount of 50 mass%, 75 mass% or more, preferably 90 mass% or more, and more preferably 100 mass%; and that fibers are heat-adhered to each other due to the sheath component of the composite fiber in the thermally bonded non-woven fabric. Specifically, the sheath component of the composite fiber is preferably softened or molten so that the fibers are firmly fixed to each other. The heat-adhesion of the sheath component by softening or melting can be achieved by heat treatment using thermal embossing rolls or hot air at a temperature that is equal to or higher than the softening point of the sheath component, and that is less than the melting point of the core component of the composite fiber.

Additionally, in an embodiment, fibers may be integrated using hydroentanglement. Hydroentanglement may be combined with the thermal bonding. The conditions for hydroentanglement are determined according to the basis weight, flexibility, and functionality of the non-woven fabric to be ultimately obtained. When forming openings in a non-woven fabric, it is preferable to determine the conditions while taking the openings into consideration. Hydroentanglement may be performed, for example, by spraying one or both surfaces of a fibrous web with a pillar water stream from a nozzle provided with orifices having a pore diameter of 0.05 to 0.5 mm at intervals of 0.5 to 1.5 mm under a water pressure of 1 to 20 MPa, one to eight times for each surface.

The fiber assembly that contains the fiber of the present invention may also contain fiber other than this fiber. The other fiber is not particularly limited. It may be, for example, natural fibers, such as cotton, silk, and wool; or recycled fibers, such as viscose rayon, cupra, and solvent-spun cellulose fiber (e.g., Lenzing Lyocell (registered trademark) and Tencel (registered trademark)). Alternatively, the other fiber may be a synthetic fiber to whose surface a fiber treatment agent other than the specific fiber treatment agent described above is adhered. Since the resins suitable for constituting the synthetic fiber and the forms of the synthetic fiber are as explained above, the explanation concerning such resins and forms of synthetic fiber is omitted here. These fibers may be used singly, or in a combination of two or more.

When the fiber assembly according to the present invention is prepared in the form of non-woven fabric for use in the top sheet (surface material) of a hygiene product, the non-woven fabric is preferably a heat-adhesive non-woven fabric. In other words, a surface material of hygiene products preferable for use is a heat-adhesive non-woven fabric obtained by preparing a fibrous web with a desired basis weight using only the synthetic fiber according to the present invention or a mixture of the synthetic fiber according to the present invention with another fiber, using, for example, a carding or air-laid technique; and optionally subjecting the fibrous web to entangling treatment to allow fibers to adhere to each other by heat. The non-woven fabric may have a laminated structure formed of a fibrous web that contains the fiber according to the present invention (or a fibrous web that consists of the fiber according to the present invention) and a fibrous web formed of another fiber. In any case of structure, the surface material of hygiene products contains the fiber according to the present invention in an amount of 50 mass% or more, 75 mass% or more, preferably 90 mass% or more, and more preferably 100 mass%. This enables the body fluid of humans or animals to quickly move from the body to the absorber.

The basis weight of the fiber assembly that contains the fiber according to the present invention is not particularly limited, and can be suitably selected according to the intended use. For example, the fiber assembly according to the present invention in the form of non-woven fabric for use in the surface material of hygiene products preferably has a basis weight of 10 to 80 $g/m^2$. The fiber assembly according to the present invention in the form of non-woven fabric for

use in various wipes in sheet form, such as pre-moistened wipes, wipes, and disposable hand towels, for humans, animals, or items preferably has a basis weight of 20 to 100 g/$m^2$. The fiber assembly according to the present invention in the form of non-woven fabric for use in cosmetic-impregnated sheets or cosmetic or medical patches, such as facial masks, preferably has a basis weight of 20 to 200 g/$m^2$.

When a fiber assembly that contains the fiber according to the present invention is provided as a non-woven fabric, the non-woven fabric is preferably incorporated in a skin-contacting product. Specifically, the present invention also provides a skin-contacting product at least part of which is the non-woven fabric described above. As used herein, the phrase "skin-contacting product" refers to a product used by bringing it into contact with the skin of humans or non-human animals. Specifically, skin-contacting products include the following.

· Body fluid-absorbing products (specifically, paper diapers for babies, paper diapers for adults, sanitary napkins, pantyliners, incontinence pads, interlabial pads, nursing pads, sweat-absorbing sheets, animal excrement treatment materials, paper diapers for animals, urine absorption sheets for animals, etc.)

· Skin-covering sheets (specifically, base cloth of cosmetic or medical patches such as facial masks, and cooling or warming plasters, and base cloth of wound surface protection sheets, non-woven fabric bandages, hemorrhoid pads, heating items that directly come in contact with the skin (e.g., disposable body warmers), various patches for animals, etc.)

· Wipes for humans (makeup remover wipes, antiperspirant wipes, wipes for the buttocks, etc.), various wipes in sheet form for animals, etc.

· Others (e.g., disposable clothing, such as disposable underwear and medical gowns, masks, wound protection clothing for animals, bandage fiber portions, bandages, medical gauze, etc.)

A non-woven fabric that contains the fiber according to the present invention may be part of these products, or may constitute the entire product. For example, a non-woven fabric that contains the fiber according to the present invention may constitute only the surface material of a body fluid-absorbing product. A non-woven fabric that contains the fiber according to the present invention may constitute only the part that covers the sensitive part in a skin-covering sheet, or the entire skin-covering sheet.

Examples

The following describes the present invention in detail with reference to Examples. However, the present invention is not limited to the Examples.

Preparation of Fiber Treatment Agent

The substances listed below used as component (a) and component (b) were mixed in the ratios illustrated in Table 1 (mass%), thereby preparing fiber treatment agents.

Component (a)

a-1: mannosylerythritol lipid B (trade name: Ceramela (registered trademark), produced by Toyobo Co., Ltd.)
a-2: sodium surfactin (trade name: KANEKA Surfactin, produced by Kaneka Corporation)
a-3: sophorolipid (trade name: ACS-Sophor, produced by Allied Carbon Solutions Co., Ltd.)

Component (b)

b-1: decaglycerol monolaurate (trade name: ML750, produced by Sakamoto Yakuhin Kogyo Co., Ltd.)
b-2: polyoxypropylene diglycerol ether (the average number of moles of propylene oxide added: 14) (trade name: SC-P1000, produced by Sakamoto Yakuhin Kogyo Co., Ltd.)
b-3: polyglycerol (an oil agent that contains about 25 mass% of polyglycerol (trade name: TES8327, produced by Takemoto Oil & Fat Co., Ltd) was used as a polyglycerol supply source)

Example 1

High-density polyethylene (trade name: Nipolon Hard OS02H, produced by Tosoh Corporation) that has a melting point of 130°C before spinning, a melt flow rate of 20 g/10 min as measured in accordance with JIS K6922-1 at a temperature of 190°C in a mass of 2.16 kgf, and a density of 0.952; and polyethylene terephthalate (trade name: HY-01, produced by Hengyi) that has a melting point of 260°C before spinning and an intrinsic viscosity (IV value) of 0.640 dl/g, were used. From the spinning nozzle of an ejecting hole (pore diameter $\phi$: 0.35), the polyethylene terephthalate resin and the polyethylene resin were spun in a mass ratio of the core component to the sheath component (core component:sheath component) of 60/40, followed by melt-spinning at a take-up speed of 1200 m/in, thereby obtaining a undrawn core-in-sheath composite yarn with a fineness of 5.4 dtex.

Subsequently, this undrawn yarn was subjected to wet-drawing treatment at a drawing temperature of 80°C in a 2.67-fold draw ratio, thereby forming drawn yarn. A treatment agent prepared by mixing two types of components in the proportions illustrated in the row of Example 1 of Table 1 was diluted with water such that the treatment agent had a concentration of 8.0 mass%, thereby preparing a treatment solution. The treatment solution was added to the drawn yarn with a roller lubricator, and then crimps were added with a fiber-crimping machine (crimper). The yarn was subjected to a drying step at a drying temperature of 100°C for 15 minutes to evaporate and dry out the moisture in the surface of fibers; and cut to a fiber length of 44 mm with a cutter, thereby obtaining core-in-sheath composite fibers that have a fineness of 2.4 dtex, a fiber length of 44 mm, a number of crimps of 18/25 mm, and a crimp percentage of 16%.

The obtained fibers were formed into a fibrous web with a basis weight of 30 g/m$^2$ using a roller card; and the sheath component of the fibers was melted at a heating treatment temperature of 140°C with a hot-air spraying machine to allow the fibers of the fibrous web to be heat-adhered to each other, thereby obtaining a heat-adhesive non-woven fabric.

Examples 2 to 7, Comparative Examples 1 to 6, Reference Examples 1 to 3

The procedure of Example 1 was repeated except that as a fiber treatment agent, those prepared by mixing the components in the proportions shown in the rows of Examples 2 to 7, Comparative Examples 1 to 6, and Reference Examples 1 to 3 of Table 1 were used, thereby obtaining fibers. Heat-adhesive non-woven fabrics were prepared from the fibers, and the amount of each treatment agent to the surface of the fibers was determined by the method described above. Table 1 illustrates the results. In Examples 6 and 7, a fiber treatment agent was prepared using an oil agent containing 25 mass% of polyglycerol as a polyglycerol of b-3. The proportion of component (b) and the amount of adhered component (b) shown in Table 1 are based on the amount of polyglycerol, not the amount of the oil agent.

Measurement of the Amount of the Adhered Fiber Treatment Agent

The percentage of the treatment agent adhered to the surface of the fibers was measured by a rapid extraction method using a rapid residual fat extractor (Soxtec™ 2055, produced by Foss). First, fibers that were cut to a predetermined length, and that were treated with a fiber treatment were dried with a hot-air dryer (105°C×30 minutes), and treated with a fiber spreader (opener) twice. Eight grams of raw cotton (Wf) was weighed and placed in a metallic cylinder (inner diameter: 35 mm, length: 75 mm, bottom: 100-mesh plain weave-metallic gauze filter), followed by immersing the cylinder in 90 ml of a solvent (ethanol/hexane = 75/25) in an aluminum cup. The aluminum cup (mass: $W_{tray}$) that contained the solvent (ethanol/hexane) in which the treatment agent adhered to the fiber sample was dissolved was heated to evaporate the solvent. Before the solvent was placed in the aluminum cup, the aluminum cup was sufficiently dried (105°C×10 minutes) with a dryer, and the mass ($W_{tray}$) of the aluminum cup was measured. After the solvent was completely evaporated, the mass ($W_{fat}$) of the aluminum cup in which the fiber treatment agent remained was measured. After the measurement, the amount of the treatment agent adhered to the surface of fibers relative to the mass of fibers was calculated from the following formula. The amount of the treatment agent adhered to the surface of fibers of Example 1 was measured by this method; the amount of the adhered treatment agent was 0.5 mass% based on the mass of fibers. The amount of the adhered treatment agent was multiplied by the content (mass%) of component (a) and component (b) in the fiber treatment agent, thereby determining the amount (mass%) of adhered component (a) and the amount (mass%) of adhered component (b) based the mass of fibers. Table 1 illustrates the results.

**Formula for Calculating the Amount of Adhered Treatment Agent**

$$\text{The Amount of Adhered Treatment Agent (\%)} = \frac{W_{fat}\ \text{(Mass after Extraction)} - W_{tray}\ \text{(Mass before Extraction)}}{\text{Mass of Sample } (W_f)} \times 100$$

Evaluation of Hydrophilicity

The obtained non-woven fabrics of the Examples and Comparative Examples were evaluated for hydrophilicity. The evaluation of hydrophilicity was performed by using a RUN-OFF test (a method called "EDNA RUN-OFF test," which is recommended by the European Disposables and Nonwovens Association (EDNA)); specifically, in accordance with the following procedure. First, a non-woven fabric was cut into a piece 36 cm in the vertical direction (machine direction) and 15 cm in the lateral direction, thereby preparing a sample. This non-woven fabric sample was placed and fixed on a support table so that the vertical direction and the horizontal plane formed an angle of 45 degrees. At this stage, filter paper No. 5A (produced by Advantec Co., Ltd.) was laid on the slope of the support table, and five sheets of the non-woven fabric sample to be measured were stacked thereon and fixed. The support table had a cross-section of substantially an isosceles right triangle that had a 45-degree angle to the horizontal plane. From a height of 1 cm, at a position of 1 cm from the upper end of the surface of the non-woven fabric, a total of 10 g of blue-colored physiological saline was added dropwise with a burette at a rate of 2 g/10 seconds. After all of the dropped physiological saline was absorbed by the non-woven fabric, the position on the non-woven fabric from which the water droplets of the physiological saline disappeared was measured. The distance over which the water droplets ran off between the position above and the position at which physiological saline was dropped on the surface of the non-woven fabric was determined. In each Example, the test was performed 10 times using 10 samples. Table 1 illustrates the average (mm) of runoff values (the distance over which the droplets ran off).

The shorter the distance, the higher the hydrophilicity of the non-woven fabric. More specifically, a non-woven fabric that shows a shorter distance is considered to have a higher ability to instantaneously absorb water. On the other hand, a non-woven fabric with a low hydrophilicity hardly absorbs dropped physiological saline, and this distance becomes longer.

Evaluation of Moisturizing Properties

The obtained non-woven fabrics of the Examples, Comparative Examples, and Reference Examples were evaluated for moisturizing properties. Five subjects (males and females in their 30s to 40s) participated in the evaluation. The inner side of the forearm of the subjects was washed with a commercially available detergent (trade name: CHARMY V Quick, produced by Lion Corporation) five times. After the subjects were allowed to rest at 22°C at 50% RH for 30 minutes, the moisture content in the keratinous layer of the inner side of the forearm washed with the commercially available detergent was measured with a Corneometer CM825 (Courage + Khazaka electronic GmbH). The obtained non-woven fabrics of the Examples, Comparative Examples, or Reference Examples (sample size: 3 cm × 3 cm) were then adhered to the inner side of the forearms on which the moisture content in the keratinous layer was measured. After 120 minutes with the non-woven fabrics adhered to the forearms, the moisture content in the keratinous layer was measured. The moisture content in the keratinous layer before a non-woven fabric was adhered was deducted from the moisture content in the keratinous layer after the non-woven fabric was adhered, in order to determine the increase in the moisture content in the keratinous layer. Table 1 illustrates the results.

Table 1

| | Formulation of Fiber Treatment Agent | | | | Amount Adhered to Fiber | | | Hydrophilicity | Moisturizing Properties |
|---|---|---|---|---|---|---|---|---|---|
| | Component (a) | | Component (b) | | Adhered Amount based on Mass of Fiber (mass%) | | | Runoff Value (mm) | Increase in Moisture Content in Keratinous Layer (a.u.) |
| | Type | Proportion (mass%) | Type | Proportion (mass%) | Treatment Agent | Component (a) | Component (b) | | |
| Example 1 | a-1 | 33.3 | b-1 | 66.7 | 0.5 | 0.167 | 0.334 | 12.9 | 1.35 |
| Example 2 | a-1 | 66.7 | b-1 | 33.3 | 0.5 | 0.334 | 0.167 | 16.6 | 1.49 |
| Example 3 | a-1 | 33.3 | b-1 | 66.7 | 1 | 0.333 | 0.667 | 10.8 | 1.44 |
| Example 4 | a-1 | 33.3 | b-2 | 66.7 | 0.5 | 0.167 | 0.334 | 22.3 | 1.30 |
| Example 5 | a-1 | 66.7 | b-2 | 33.3 | 0.5 | 0.334 | 0.167 | 25.5 | 1.48 |
| Example 6 | a-1 | 33.3 | b-3 | 16.7 | 0.5 | 0.167 | 0.083 | 15.6 | 1.34 |
| Example 7 | a-1 | 66.7 | b-3 | 8.3 | 0.5 | 0.334 | 0.042 | 13.0 | 1.48 |
| Reference Example 1 | - | 0 | b-1 | 100 | 0.333 | 0 | 0.333 | 20.2 | 0.22 |
| Reference Example 2 | a-1 | 100 | | 0 | 0.167 | 0.167 | 0 | 193.1 | 1.35 |
| Reference Example 3 | - | 0 | b-1 | 100 | 0.167 | 0 | 0.167 | 30.8 | 0.15 |
| Comparative Example 1 | a-2 | 33.3 | b-1 | 66.7 | 0.5 | 0.167 | 0.334 | 91.3 | 0.20 |
| Comparative Example 2 | a-3 | 33.3 | b-1 | 66.7 | 0.5 | 0.167 | 0.334 | 37.0 | 016 |
| Comparative Example 3 | a-2 | 66.7 | b-1 | 33.3 | 0.5 | 0.334 | 0.167 | 104.6 | 0.23 |
| Comparative Example 4 | a-3 | 66.7 | b-1 | 33.3 | 0.5 | 0.334 | 0.167 | 200.3 | 0.21 |
| Comparative Example 5 | a-2 | 66.7 | b-2 | 33.3 | 0.5 | 0.334 | 0.167 | 155.9 | 0.19 |
| Comparative Example 6 | a-3 | 66.7 | b-2 | 33.3 | 0.5 | 0.334 | 0.167 | 157.6 | 0.24 |

Examples 1 to 7 exhibited a low runoff value and excellent hydrophilicity, while exhibiting moisturizing properties equivalent to or greater than those of Reference Example 2; with the moisturizing properties of component a-1 (MEL) not being impaired. From the low runoff value of 20.2 in Reference Example 1, the glycerol compound was confirmed to have hydrophilicity-imparting action. However, from the high runoff value of 193.1 in Reference Example 2, component a-1 (MEL) alone was confirmed to have almost no hydrophilicity-imparting action. Nonetheless, a comparison between Example 1 and Reference Example 1 indicates that the use of component a-1 (MEL) and a glycerol compound in combination results in a runoff value lower than that of the use of a glycerol compound alone (Reference Example 1), and increases the hydrophilicity of the fibers. Thus, the use of a glycerol compound in combination with MEL was confirmed to not only impart moisturizing properties inherent to MEL to fibers, but also increase the hydrophilicity-imparting action of glycerol.

Comparatively, a comparison between Reference Example 1 and Comparative Example 1 or 2 indicates that the use of component a-2 (sodium surfactin) or component a-3 (sophorolipid), which are other biosurfactants, resulted in a runoff value higher than the runoff value of the glycerol compound (20.2); and was thus confirmed to decrease the hydrophilicity-imparting action of the glycerol compound.

## Claims

**1.** A synthetic fiber comprising a fiber treatment agent adhered to the surface of the fiber,

wherein the fiber treatment agent contains (a) a mannosylerythritol lipid (MEL), and (b) at least one polyglycerol compound selected from the group consisting of polyglycerols, polyglycerol fatty acid esters, and alkylene oxide adducts of polyglycerols,
the total mass of component (a) and component (b) is 40 mass% or more based on the entire fiber treatment agent, and
the mass of component (a) is 33 to 99.5 mass% based on the total mass of component (a) and component (b), wherein the fiber treatment agent contains substantially no lactate.

**2.** The synthetic fiber according to claim 1, wherein the MEL is at least one member selected from the group consisting of mannosylerythritol lipid A (MEL-A), mannosylerythritol lipid B (MEL-B), mannosylerythritol lipid C (MEL-C), mannosylerythritol lipid D (MEL-D), triacylated MEL-A, triacylated MEL-B, triacylated MEL-C, and triacylated MEL-D.

**3.** A fiber assembly comprising the synthetic fiber of claim 1 or 2 in an amount of 50 mass% or more.

**4.** A skin-contacting product comprising the fiber assembly of claim 3 in a skin-contacting part of the skin-contacting product, the fiber assembly being a non-woven fabric.

**5.** The skin-contacting product according to claim 4, which is a hygiene product.

**6.** A fiber treatment agent comprising

(a) a mannosylerythritol lipid (MEL), and
(b) at least one polyglycerol compound selected from the group consisting of polyglycerols, polyglycerol fatty acid esters, and alkylene oxide adducts of polyglycerols,

wherein component (a) is present in an amount of 33 to 99.5 mass% based on the total mass of component (a) and component (b), and
the total mass of component (a) and component (b) is 40 mass% or more based on the entire fiber treatment agent,
wherein the fiber treatment agent contains substantially no lactate.

## Patentansprüche

**1.** Synthetische Faser, umfassend ein Faserbehandlungsmittel, das an der Oberfläche der Faser haftet,

wobei das Faserbehandlungsmittel (a) ein Mannosylerythritol-Lipid (MEL) und (b) mindestens eine Polyglycerinverbindung, ausgewählt aus der Gruppe bestehend aus Polyglycerinen, Polyglycerinfettsäureestern und

Alkylenoxidaddukten von Polyglycerinen, enthält,
wobei die Gesamtmasse der Komponente (a) und der Komponente (b), bezogen auf das gesamte Faserbehandlungsmittel, 40 Massen-% oder mehr beträgt und
die Masse der Komponente (a), bezogen auf die Gesamtmasse der Komponente (a) und der Komponente (b), 33 bis 99,5 Massen-% beträgt,

wobei das Faserbehandlungsmittel im Wesentlichen kein Lactat enthält.

**2.** Synthetische Faser nach Anspruch 1, wobei das MEL mindestens ein Mitglied ist, ausgewählt aus der Gruppe bestehend aus Mannosylerythritol-Lipid A (MEL-A), Mannosylerythritol-Lipid B (MEL-B), Mannosylerythritol-Lipid C (MEL-C), Mannosylerythritol-Lipid D (MEL-D), triacyliertem MEL-A, triacyliertem MEL-B, triacyliertem MEL-C und triacyliertem MEL-D.

**3.** Faseranordnung, die die synthetische Faser nach Anspruch 1 oder 2 in einer Menge von 50 Massen-% oder mehr enthält.

**4.** Hautberührendes Produkt, umfassend die Faseranordnung nach Anspruch 3 in einem hautberührenden Teil des hautberührenden Produkts, wobei die Faseranordnung ein Vliesstoff ist.

**5.** Hautberührendes Produkt nach Anspruch 4, welches ein Hygieneprodukt ist.

**6.** Faserbehandlungsmittel, umfassend

(a) ein Mannosylerythritol-Lipid (MEL), und
(b) mindestens eine Polyglycerinverbindung, ausgewählt aus der Gruppe bestehend aus Polyglycerinen, Polyglycerinfettsäureestern und Alkylenoxidaddukten von Polyglycerinen,
wobei die Komponente (a) in einer Menge von 33 bis 99,5 Massen-%, bezogen auf die Gesamtmasse der Komponente (a) und der Komponente (b), vorhanden ist und
die Gesamtmasse von Komponente (a) und Komponente (b), bezogen auf das gesamte Faserbehandlungsmittel, 40 Massen-% oder mehr beträgt,

wobei das Faserbehandlungsmittel im Wesentlichen kein Lactat enthält.

## Revendications

**1.** Fibre synthétique qui comprend un agent de traitement des fibres qui adhère à la surface de la fibre ;

dans lequel l'agent de traitement des fibres contient (a) un lipide de mannosylérythritol (MEL), et (b) au moins un composé de polyglycérol qui est choisi parmi le groupe constitué par des polyglycérols, des esters d'acides gras de polyglycérol et des adduits d'oxydes d'alkylènes de polyglycérols ;
la masse totale du composant (a) et du composant (b) s'élève à 40 % en masse ou plus, basés sur l'agent de traitement des fibres dans son entièreté ; et
la masse du composant (a) s'élève de 33 à 99,5 % en masse, basés sur la masse totale du composant (a) et du composant (b) ;
dans laquelle l'agent de traitement des fibres ne contient essentiellement aucun lactate.

**2.** Fibre synthétique selon la revendication 1, dans laquelle le MEL représente au moins un membre qui est choisi parmi le groupe constitué par un lipide de mannosylérythritol A (MEL-A), un lipide de mannosylérythritol B (MEL-B), un lipide de mannosylérythritol C (MEL-C), un lipide de mannosylérythritol D (MEL-D), un MEL-A triacétylé, un MEL-B triacétylé, un MEL-C triacétylé, et un MEL-D triacétylé.

**3.** Assemblage de fibres qui comprend la fibre synthétique selon la revendication 1 ou 2 en une quantité de 50 % en masse ou plus.

**4.** Produit entrant en contact avec la peau qui comprend l'assemblage de fibres de la revendication 3 dans une partie entrant en contact avec la peau du produit entrant en contact avec la peau, l'assemblage de fibres représentant un non-tissé.

**5.** Produit entrant en contact avec la peau selon la revendication 4, à savoir un produit d'hygiène.

**6.** Agent de traitement des fibres qui comprend :

(a) un lipide de mannosylérythritol (MEL) ; et
(b) au moins un composé de polyglycérol qui est choisi parmi le groupe constitué par des polyglycérols, des esters d'acides gras de polyglycérol et des adduits d'oxydes d'alkylènes de polyglycérols ;
dans lequel le composant (a) est présent en une quantité de 33 à 99,5 % en masse, basés sur la masse totale du composant (a) et du composant (b) ; et
la masse totale du composant (a) et du composant (b) s'élève à 40 % en masse ou plus, basés sur l'agent de traitement des fibres dans son entièreté ;
dans lequel l'agent de traitement des fibres ne contient essentiellement aucun lactate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012057275 A
- WO 2016076089 A